# EUROPEAN PATENT SPECIFICATION

(11) **EP 0 450 491 B1**
(45) Date of publication and mention of the grant of the patent: **10.01.1996**
(21) Application number: 91104874.2
(22) Date of filing: 27.03.1991
(51) Int. Cl.: C12P 7/48

(54) **Process for producing d-isocitric acid by fermentation**
Verfahren zur Herstellung von D-Isocitronensäure durch Fermentation
Procédé pour la production d'acide D-isocitrique par fermentation

(30) Priority: 03.04.1990 JP 88876/90
(43) Date of publication of application: 09.10.1991
(73) Proprietor: KYOWA HAKKO KOGYO CO., LTD., Chiyoda-ku, Tokyo-to (JP)
(72) Inventor: Kino, Kuniki, Yamaguchi-ken (JP); Tomiyoshi, Yasuhiro, Yamaguchi-ken (JP); Kuratsu, Yoshiyuki, Yamaguchi-ken (JP)
(74) Representative: VOSSIUS & PARTNER

(56) References cited:
- DE-A- 2 264 764
- GB-A- 1 334 220
- US-A- 3 801 455
- AGRICULTURAL AND BIOLOGICAL CHEMISTRY. vol. 36, no. 2, 1972, TOKYO JP pages 339 - 341; AKIYAMA, S. ET AL.: 'Production of citric acid from n-paraffins by fluoroacetate sensitive mutant strains of Candida lipolytica.'
- CHEMICAL ABSTRACTS, vol. 81, no. 13, 30 September 1974, Columbus, Ohio, US; abstract no. 76450Q, TABUCHI, T. & HARA, S.: 'Production of 2-methylisocitric acid from n-paraffins by mutants of Candida lipolytica.' page 349 ;column 2 ;
- WORLD PATENTS INDEX LATEST Section Ch, Week 9004, Derwent Publications Ltd., London, GB; Class C, AN 90-028516

## Description

The present invention relates to a process for producing d-isocitric acid by fermentation, and more particularly a process for producing d-isocitric acid using a microorganism belonging to the genus Candida, having resistance to monofluoroacetic acid, and being incapable of growing or capable of growing only poorly in the presence of d-isocitric acid as a sole carbon source.

d-Isocitric acid is useful as a medicament, a food additive, and the like.

Heretofore, a process for producing d-isocitric acid by fermentation using yeast has been known [Nippon Nôgeikagaku Kaishi(Journal of Japan Society for Bioscience, Biotechnology and Agrochemistry) , 44(11): 493-498(1970); British Patent No. 1,199,700, French Patent No. 1,596,056]. GB-A-1334220 discloses a process for the production of a mixture of citric acid and isocitric acid by fermentation which comprises culturing of an appropriate yeast strain. By the addition of monofluoroacetic acid, the yield of citric acid could be increased, whereas the yield of isocitric acid considerably decreased.

An attempt has previously been made to produce a fluoroacetic acid-resistant mutant from Candida lipolytica in order to obtain a strain having high productivity in d-isocitric acid. In this attempt, a mutant was obtained which exhibits increased aconitase activity, but said mutant could not produce an increased level of d-isocitric acid [Nippon Nôgeikagaku Kaishi (Journal of Japan Society for Bioscience, Biotechnology and Agrochemistry), 48(10) : 543-548, 549-554(1974)].

Further, there are known processes using yeast for fermentation in which itaconic acid is added for improving the yield of d-isocitric acid (Summary of Lectures at 1976 General Meeting of Japan Society for Bioscience, Biotechnology and Agrochemistry, p 348; Japanese Published Examined Patent Application No. 39194/1981) and a process using Candida zeylanoides, in which potassium ferrocyanide is added (J. Ferment. Technol., 52(8): 542-550 (1974)) .

However, these known processes are not satisfactory in productivity of d-isocitric acid, and it has been desired to develop a process for industrially producing d-isocitric acid at a low cost.

The present invention provides a process for producing d-isocitric acid, which comprises culturing in a medium a microorganism until d-isocitric acid is accumulated in the culture and recovering d-isocitric acid therefrom, said microorganism belonging to the genus Candida, having resistance to monofluoroacetic acid, being incapable of growing or capable of growing only poorly in the presence of d-isocitric acid as a sole carbon source, and being capable of producing d-isocitric acid.

Any microorganism can be used in the present invention so long as it belongs to the genus Candida which is resistant to monofluoroacetic acid, incapable of growing or capable of growing only poorly in the presence of d-isocitric acid as a sole carbon source, and capable of producing d-isocitric acid. Examples of the microorganisms are those of the species Candida lipolytica, Candida zeylanoides, Candida guilliermondii, Candida albicans, Candida humicola, Candida parapsilosis, and Candida brumptii. Candida zeylanoides H-7728 is a preferred strain, which was deposited with the Fermentation Research Institute, Agency of Industrial Science and Technology(FRI), Japan, on February 16, 1990, with accession number FERM BP-2756.

Either a wild type microorganism or a mutant can be used in the present invention provided that said microorganism or the mutant has the above-mentioned characteristics.

A mutant can be obtained by known methods such as UV radiation and treatment with chemicals such as N-nitro-N'-methyl-N-nitrosoguanidine(NTG).

D-isocitric acid can be obtained by culturing one of the above microorganisms in a conventional manner in a synthetic medium or a natural medium containing carbon sources, nitrogen sources, inorganic compounds, growth factors and the like until d-isocitric acid is accumulated in the culture, and recovering d-isocitric acid therefrom.

As the carbon sources, carbohydrates such as glucose, sucrose, molasses and starch hydrolyzates; alcohols such as ethanol and glycerol; organic acids such as acetic acid; aliphatic acids such as oleic acid; fats and oils such as soybean oil and fish oil; paraffins such as tetradecane and hexadecane; etc. can be used. Furthermore, organic compounds can also be used depending upon the assimilability of the microorganism to be used.

As the nitrogen sources, inorganic and organic ammonium compounds such as ammonia, ammonium chloride, ammonium sulfate, ammonium carbonate and ammonium acetate; nitrogenous compounds such as urea and other nitrogen-containing compounds; nitrogen-containing organic materials such as peptone, meat extract, yeast extract, corn steep liquor and casein hydrolyzate; etc. can be used. Nitrates can also be used as a nitrogen source depending on the microorganism to be used. The above mentioned nitrogen sources can be used alone or in combination.

As the inorganic compounds, dipotassium hydrogen phosphate, potassium dihydrogen phosphate, ammonium sulfate, ammonium chloride, magnesium sulfate, sodium chloride, ferrous sulfate, manganese sulfate, calcium carbonate, etc. can be used.

Vitamins, amino acids, etc. necessary for the growth of the microorganisms are also added to the medium. It is not necessary to add them when they are contained in the other components mentioned above.

Culturing is carried out under aerobic conditions by shaking culture or aeration culture with stirring at a temperature of 20°C to 35°C, and the pH of the medium is maintained in the range of 2.5-10, preferably in the range of 3-8. D-isocitric acid is accumulated in the culture after 2 to 7 days. Thereafter, precipitate such as cells are removed from the culture by means of centrifugation, etc., and d-isocitric acid can be recovered from the culture supernatant by simultaneous application of ion exchange treatment, concentration, active charcoal treatment and the like.

The present invention is illustrated by the following Examples.

### Example 1 Preparation of a Mutant

Candida zeylanoides ATCC 15585 capable of producing d-isocitric acid was used as a parent strain in this Example. The strain was cultured in YM medium (3 g of malt extract, 3 g of yeast extract, 5 g of polypeptone and 10 g of glucose in 1 l of water, pH adjusted to 6.0) at 30°C for 20 hours. Cells(10⁸⁻⁹/ml) were recovered from the culture, washed with 0.2 M acetate buffer (pH 5.0) and suspended in the same buffer. NTG was added to the suspension to a final concentration of 1 mg/ml, followed by incubation at 30°C for 1 hour to induce mutagenesis. The cells thus treated were washed with the same buffer, spread on an agar plate of minimal medium [6.7 g/l Bacto Nitrogen Base®(Difco), 2% agar, pH 5.5] containing 4 g/l sodium monofluoroacetate and 1 g/l L-proline, and cultured at 30°C for 5-10 days. Colonies growing on the plate were separated as monofluoroacetic acid-resistant mutants. Among them, those incapable of growing on the minimal medium containing 1 % d-isocitric acid, or showing extremely poor growth on the medium in comparison with the parent strain were selected, and one of them was designated as Candida zeylanoides H-7728 (FERM BP-2756).

The sensitivity to monofluoroacetic acid and the growth in the presence of individual carbon sources of the mutant H-7728 are shown in Table 1 in comparison with those of the parent strain ATCC 15585.

The sensitivity to monofluoroacetic acid of the mutant H-7728 and of the parent strain ATCC 15585 was determined by spreading cells on an agar plate of the above minimal medium containing 4 g/l sodium monofluoroacetate and culturing the cells at 30°C for 7 days. The cells grown on the same medium without sodium monofluoroacetate were used as a control.

The growth in the presence of individual carbon sources was determined spectrophotometrically by measuring the absorption of the medium at 660nm after culturing cells which had been grown on YM medium, washed with 0.85% sodium chloride solution and suspended in the same solution, in a minimal medium [6.7 g/l Bacto Nitrogen Base®(Difco), pH 5.5] containing 2% calcium carbonate and 1% carbon source to be tested, with shaking at 30°C for 48 hours.

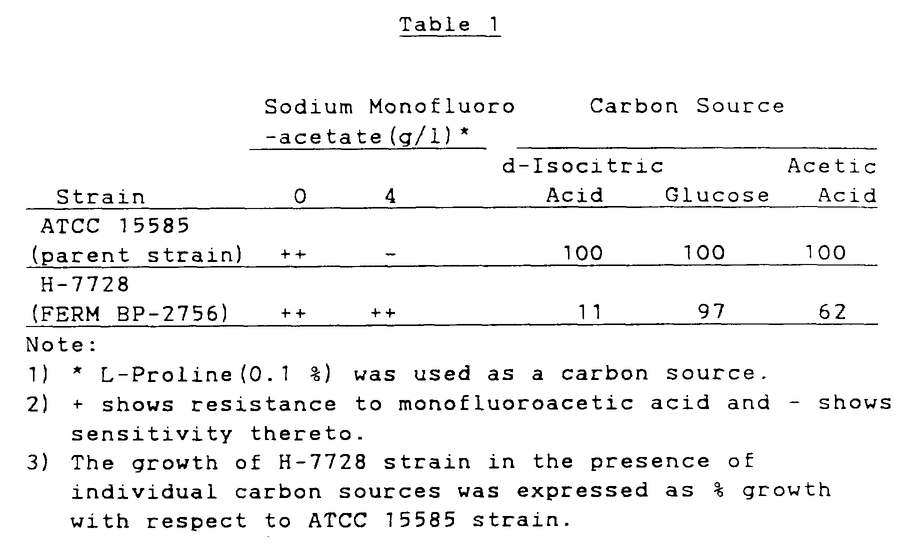

### Example 2 Production of d-Isocitric Acid

Candida zeylanoides H-7728 (FERM BP-2756) obtained in Example 1 was inoculated into 250-ml Erlenmeyer flask containing 40 ml of a seed medium (5% glucose, 0.2% NH₄Cl, 0.05% KH₂PO₄ , 0.05% HgSO₄·7H₂O, 0.1% yeast extract, 0.1% corn steep liquor, 3.3% CaCO₃, pH 5.5), and incubated at 30°C for 24 hours on a rotary shaker at 210 rpm. The obtained seed culture (4 ml) was inoculated into 250-ml Erlenmeyer flask containing 40 ml of a fermentation medium of the composition described below, and incubated for 7 days under the same conditions as in the seed culturing.

### Composition of the Fermentation Medium

5% n-paraffin (a mixture mainly composed of C₁₂- to C₁₅-paraffins, Nippon Mining), 0.4% NH₄Cl, 0.05% KH₂PO₄ , 0.05% MgSO₄·7H₂O, 2 mg/l ZnSO₄·7H₂O, 2 mg/l MnSO₄·4-6H₂O, 5 mg/l FeSO₄·7H₂O, 150 µg/l CuSO₄·5H₂O, 100 µg/l biotin, 5 mg/l thiamine hydrochloride, 3% CaCO₃, pH 5.5.

The parent strain ATCC 15585 was incubated in a similar manner as above and used as a control.

The yield of d-isocitric acid and the amount of the by-product, citric acid, were determined by high performance liquid chromatography(HPLC). Citric acid was formed as a precipitate of calcium salt thereof. The precipitate was dissolved by the addition of 0.5 N HCl, followed by centrifugation, and the resultant supernatant was subjected to HPLC.

The results are shown in Table 2.

**Table 2**

| Amount of Organic Acid Produced (g/l) | | |
|---|---|---|
| strain | d-Isocitric Acid | Citric Acid |
| ATCC 15585 (parent strain) | 22.5 | 23.2 |
| H-7728 (FERM BP-2756) | 30.0 | 18.3 |

Two liters of the culture of H-7728 strain was centrifuged, and the supernatant free from cells and impurities was deionized using 0.5 l of Diaion SK^{#} 1B®(H⁺-form) (Mitsubishi Kasei). After being adjusted to pH 3.6 by the addition of 10 N potassium hydroxide, the supernatant was decolorized by the treatment with active charcoal and concentrated under reduced pressure to about 100 ml, whereby monopotassium d-isocitrate crystallized. Yield, 56 g; 95 % purity.

Ten grams of the crystals was dissolved in 250 ml of water, deionized with 40 ml of Diaion SK^{#} 1B®(H⁺-form) (Mitsubishi Kasei) and concentrated at room temperature to give a paste containing a quantitative amount of d-isocitric acid corresponding to 8.35 g of pure d-isocitric acid. The paste was dissolved in 50 ml of water, heated at 100°C for 30 minutes and concentrated to dryness to give 7.57 g(quantitative) of lactonized d-isocitric acid.

## Claims

1. A process for producing d-isocitric acid which comprises culturing in a medium a microorganism until d-isocitric acid is accumulated in the culture, and recovering d-isocitric acid therefrom, said microorganism belonging to the genus Candida, having resistance to monofluoroacetic acid, being incapable of growing or capable of growing only poorly in the presence of d-isocitric acid as a sole carbon source, and being capable of producing d-isocitric acid.

2. The process according to claim 1, wherein said microorganism belongs to the species Candida lipolytica, Candida zeylanoides, Candida guilliermondii, Candida albicans, Candida humicola, Candida parapsilosis or Candida brumptii.

3. The process according to claim 2, wherein said microorganism is Candida zeylanoides H-7728 (FERM BP-2756).

4. A biologically pure culture of Candida zeylanoides H-7728 (FERM BP-2756).

## Patentansprüche

1. Verfahren zur Herstellung von d-Isocitronensäure, umfassend die Züchtung eines Mikroorganismus in einem Medium, bis sich d-Isocitronensäure in der Kultur angereichert hat, und Gewinnung der d-Isocitronensäure daraus, wobei der Mikroorganismus zur Gattung Candida gehört, eine Resistenz gegen Monofluoressigsäure aufweist und nicht oder kaum in Gegenwart von d-Isocitronensäure als einziger Kohlenstoffquelle wachsen kann und d-Isocitronensäure produzieren kann.

2. Verfahren nach Anspruch 1, wobei der Mikroorganismus zur Art Candida lipolytica, Candida zeylanoides, Candida guilliermondii, Candida albicans, Candida humicola, Candida parapsilosis oder Candida brumptii gehört.

3. Verfahren nach Anspruch 2, wobei der Mikroorganismus Candida zeylanoides H-7728 (FERM BP-2756) ist.

4. Biologisch reine Kultur von Candida zeylanoides H-7728 (FERM BP-2756).

## Revendications

1. Procédé de production d'acide d-isocitrique, qui comporte le fait de cultiver un micro-organisme dans un milieu, jusqu'à ce que de l'acide d-isocitrique se soit accumulé dans la culture, et le fait de récupérer l'acide d-isocitrique là-dedans, dans lequel procédé ledit micro-organisme, qui appartient au genre Candida, résiste à l'acide monofluoroacétique, ne peut pas proliférer, ou ne peut que mal proliférer, en présence d'acide d-isocitrique si celui-ci est l'unique source de carbone disponible, et peut produire de l'acide d-isocitrique.

2. Procédé conforme à la revendication 1, dans lequel ledit micro-organisme appartient à l'espèce Candida lipolytica, Candida zeylanoides, Candida guilliermondii, Candida albicans, Candida humicola, Candida parapsilosis, ou Candida brumptii.

3. Procédé conforme à la revendication 2, dans lequel ledit micro-organisme est Candida zeylanoides H-7728 (FERM BP-2756).

4. Culture biologiquement pure de Candida zeylanoides H-7728 (FERM BP-2756).
